# FASCICULE DE BREVET EUROPEEN

(11) **EP 3 634 294 B1**
(45) Date de publication et mention de la délivrance du brevet: **12.01.2022**
(21) Numéro de dépôt: 18727835.3
(22) Date de dépôt: 05.06.2018
(51) Int. Cl.: A61B 34/10, A61B 34/20, A61B 90/00

(54) **SYSTÈME D'AIDE AU GUIDAGE D'UN OUTIL ENDOVASCULAIRE ET PROGRAMME D'ORDINATEUR**
SYSTEM ZUR UNTERSTÜTZUNG DER STEUERUNG EINES ENDOVASKULÄREN INSTRUMENTS UND COMPUTERPROGRAMM
SYSTEM FOR ASSISTANCE IN GUIDING AN ENDOVASCULAR INSTRUMENT AND COMPUTER PROGRAMME

(30) Priorité: 06.06.2017 FR 1755011
(43) Date de publication de la demande: 15.04.2020
(73) Titulaire: THERENVA, 35000 Rennes (FR)
(72) Inventeur: LALYS, Florent, 35000 Rennes (FR); COLLEAUX, Mathieu, 35200 Rennes (FR); DURRMANN, Vincent, Dublin, Ireland D08 YY2C (IE); LUCAS, Antoine, 35690 Acigne (FR); GÖKSU, Cemil, 35000 Rennes (FR)
(74) Mandataire: Vidon Brevets & Stratégie
(86) Numéro de dépôt international: PCT/EP2018/064744
(87) Numéro de publication internationale: WO 2018/224485

(56) Documents cités:
- WO-A2-2006/063141
- FR-A1- 2 884 703
- FR-A1- 2 942 124
- FR-A1- 3 037 785

## Description

La présente invention concerne un système d'aide au guidage d'un outil endovasculaire dans des structures vasculaires d'une zone anatomique d'intérêt d'un patient, par exemple un membre inférieur d'un patient. Elle concerne également un procédé et un dispositif d'aide au guidage d'un outil endovasculaire dans des structures vasculaires associé.

Elle trouve une application dans le domaine des interventions endovasculaires guidées par l'image.

Les interventions endovasculaires permettent de traiter des maladies vasculaires de façon minimalement invasive. Elles consistent généralement à insérer par voie endovasculaire un dispositif médical dans le but d'interagir avec les tissus pathologiques. Des interventions endovasculaires sont notamment utilisées pour traiter les sténoses et les thromboses artérielles, via l'introduction de divers d'outils endovasculaires adaptés tels qu'un ballon ou un stent.

Contrairement aux interventions chirurgicales classiques qui nécessitent de réaliser une large ouverture du corps du patient pour accéder aux tissus d'intérêt, les interventions endovasculaires ne demandent que de fines incisions pour pouvoir insérer les outils dans la structure vasculaire. Elles présentent plusieurs avantages, notamment une augmentation du taux de succès à court terme ainsi qu'une diminution de la morbidité peropératoire et de la durée d'hospitalisation. Malgré la généralisation de ces interventions, elles restent délicates et nécessitent d'être sécurisées et fiabilisées. L'accès aux tissus pathologiques est rendu difficile par la nature de l'intervention. La manipulation et le contrôle des outils endovasculaires exigent une précision importante pour favoriser la réussite du traitement. Par ailleurs, le suivi des gestes opératoires ne peut être réalisé que par l'intermédiaire d'une imagerie peropératoire.

Dans le domaine des interventions endovasculaires on utilise des images bidimensionnelles acquises par fluoroscopie, permettant de guider l'insertion de dispositifs médicaux, tels des cathéters, dans l'artère fémorale et dans d'autres ramifications vasculaires.

La fluoroscopie désigne une technique d'imagerie médicale utilisant des rayons X qui permet de visualiser des structures anatomiques en mouvement et en temps réel, en particulier des structures osseuses.

Les artères étant des tissus mous et donc non visibles aux rayons X, on administre au patient un produit de contraste radio-opaque afin d'opacifier la structure vasculaire et de visualiser le cheminement des artères. On peut alors obtenir des images exploitables. Cette technique d'acquisition d'images des structures vasculaires, en particulier des artères, d'un patient est connue sous le nom d'angiographie, et les images obtenues sont appelées images angiographiques.

Les images fluoroscopiques et angiographiques bidimensionnelles sont acquises et utilisées pendant une phase opératoire, donc une phase d'intervention.

En général, les salles d'opération conventionnelles sont équipées d'un dispositif de support d'un dispositif d'imagerie à rayons X, connu sous le nom d'arceau ou de dispositif C-arm, mobile, et permettant d'acquérir des images fluoroscopiques et angiographiques avec un champ de vision donné.

Dans le cas particulier des procédures endovasculaires pour traiter l'artériopathie oblitérante des membres inférieurs, il existe un intérêt d'obtenir des images de la totalité d'un membre inférieur du patient. Or le champ de vision d'un dispositif d'acquisition d'images étant limité, il est nécessaire d'acquérir une pluralité d'images fluoroscopiques et angiographiques, de préférence partiellement superposées, pour obtenir par traitement d'images une image composite ou panoramique d'une zone anatomique d'intérêt, par exemple d'un membre inférieur du patient dans sa totalité.

De plus, dans le cadre des interventions endovasculaires conventionnelles, pour traitement des membres inférieurs, si les occlusions artérielles sont de grande taille ou occultées, elles ne peuvent pas être visualisées sur une seule image angiographique, donc plusieurs captures d'images angiographiques sont nécessaires, dans un premier temps pour visualiser l'ensemble des lésions à traiter, puis, dans un deuxième temps, pour effectuer précisément le traitement de chaque lésion.

Cependant, chaque acquisition d'image angiographique nécessite l'injection d'un produit de contraste, qui est toxique pour le patient. Par exemple, un produit de contraste iodé est néphrotoxique.

De plus, chaque acquisition d'image supplémentaire expose le patient et le personnel médical présent en salle d'opération à une quantité de rayonnement X supplémentaire.

WO 2006/063141 A2 divulgue un système d'aide au guidage selon le préambule de la revendication 1.

L'invention a pour objet de remédier aux inconvénients des méthodes connues; pour améliorer les interventions endovasculaires guidées par l'image tout en limitant le nombre d'acquisitions d'images angiographiques.

A cet effet, l'invention propose un système d'aide au guidage d'un outil endovasculaire dans des structures vasculaires d'une zone anatomique d'intérêt d'un patient, comprenant un dispositif d'imagerie apte à acquérir des images bi-dimensionnelle de portions du corps d'un patient, un dispositif programmable et une unité de visualisation, selon la revendication 1.

Avantageusement, le système de l'invention comprend l'affichage d'une zone d'image correspondant à une image fluoroscopique courante dans une image augmentée comprenant une représentation des structures vasculaires de la portion de la zone anatomique d'intérêt visualisable dans l'image fluoroscopique courante, sans nécessité de ré-injecter du produit de contraste radio-opaque dans le corps du patient.

Le système selon l'invention peut également présenter une ou plusieurs des caractéristiques

Selon un autre aspect, l'invention concerne un programme d'ordinateur selon la revendication 7.

D'autres caractéristiques et avantages de l'invention ressortiront de la description qui en est donnée ci-dessous, à titre indicatif et nullement limitatif, en référence aux figures annexées, parmi lesquelles :
- la figure 1 représente schématiquement un système d'intervention endovasculaire guidée par l'image;
- la figure 2 est un synoptique des principaux blocs d'un dispositif programmable apte à mettre en œuvre le procédé de l'invention ;
- la figure 3 est un organigramme des principales étapes d'un procédé d'aide au guidage d'un outil endovasculaire selon l'invention ;
- la figure 4 est un organigramme des principales étapes d'un procédé selon un premier mode de réalisation ;
- la figure 5 est un organigramme des principales étapes d'un procédé selon un deuxième mode de réalisation ;
- la figure 6 est un exemple d'affichage des première et deuxième images augmentées selon un mode de réalisation ;
- la figure 7 est un exemple d'affichage en superposition d'informations fluoroscopiques et angiographiques ;
- la figure 8 est un exemple de représentation 3D des structures osseuse et vasculaire.

L'invention est décrite ci-après plus particulièrement pour une aide au guidage d'outil endovasculaire dans les membres inférieurs, mais l'invention s'applique plus généralement à d'autres zones anatomiques d'intérêt, ou à une zone plus large incluant également tout ou partie du tronc du patient.

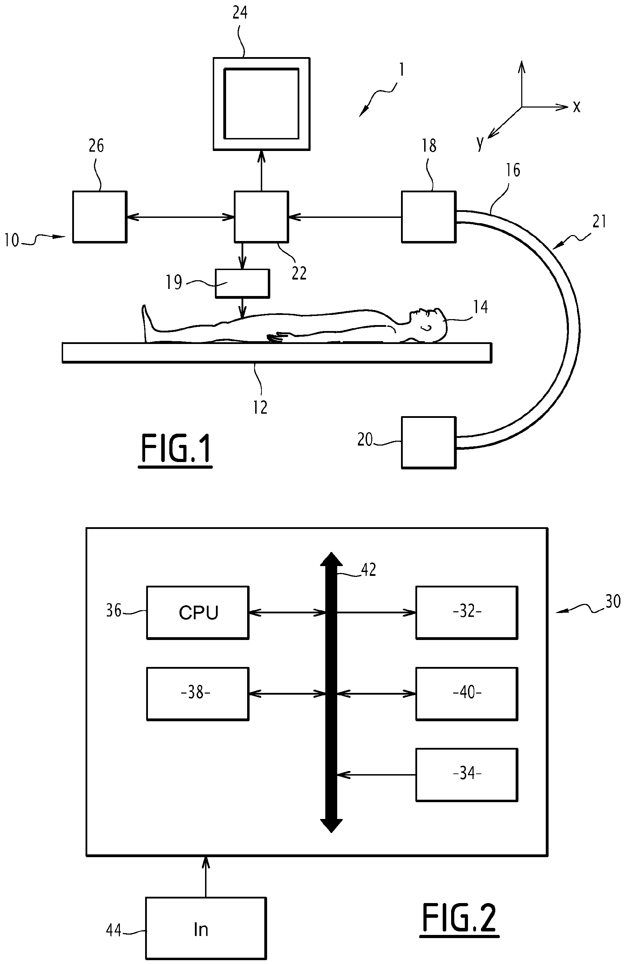

La figure 1 illustre schématiquement une salle d'opération 1, équipée d'un système 10 d'intervention endovasculaire guidée par l'image.

La salle d'opération 1 est équipée d'une table d'opération 12, sur laquelle est représenté un patient 14 à traiter par une intervention endovasculaire.

Le système d'intervention 10 comporte un dispositif 21 d'imagerie à rayon X, lui-même composé d'un dispositif de support 16 en forme d'arceau, d'une source 18 de rayons X et d'une unité 20 de réception et de détection de rayons X, positionnée en regard de la source 18. Ce dispositif d'imagerie est apte à capter des images des éléments positionnés entre la source 18 de rayons X et l'unité 20 de réception et de détection, et est également apte à tourner autour de deux axes selon le besoin de l'opérateur.

On appelle généralement les images bidimensionnelles captées par un système d'imagerie à rayons X des images fluoroscopiques.

Ainsi, le dispositif d'imagerie 21 illustré est adapté pour capter des images bidimensionnelles fluoroscopiques de diverses zones anatomiques d'intérêt du corps du patient, comprenant des structures vasculaires ciblées, en particulier des artères.

En particulier, l'opérateur peut déplacer le dispositif d'imagerie 21 sensiblement en translation le long des jambes du patient 14, pour acquérir une pluralité de N d'images fluoroscopiques Fᵢ.

Plus généralement, l'opérateur peut déplacer le dispositif d'imagerie 21 sensiblement en translation pour obtenir une pluralité d'images fluoroscopiques d'une zone anatomique d'intérêt choisie ou s'étendant du tronc supra-aortique aux pieds du patient.

Alternativement, le déplacement en translation du dispositif d'imagerie 21 est commandé, par exemple par le dispositif programmable 22, décrit ci-après.

Le nombre N d'images à acquérir est choisi en fonction du champ de vision du dispositif d'imagerie et de la taille du patient 14. Par exemple, 3 à 4 images de chaque jambe du patient 14 sont acquises le long de l'axe fémoral.

Le dispositif d'imagerie est positionné à des positions successives telles que deux images acquises successivement présentent une superposition partielle sur une zone de superposition rectangulaire, de taille prédéterminée ou variable.

De préférence, la zone de superposition a une surface supérieure ou égale à 20% de la surface des images bidimensionnelles acquises.

Le dispositif d'imagerie 21 est adapté à acquérir également des images angiographiques Aᵢ de la jambe ou des jambes du patient, suite à une injection de produit de contraste radio-opaque par une unité d'injection 19.

Comme il sera expliqué plus en détail par la suite, dans un mode de réalisation de l'invention, lors d'une première phase opératoire de préparation, à chaque position successive du dispositif d'imagerie, une image fluoroscopique et une image angiographique correspondantes sont acquises et mémorisées sous forme d'images numériques bidimensionnelles.

Le système d'intervention 10 comporte également un dispositif programmable 22, comportant un ou plusieurs processeurs, associé à une unité de visualisation 24 composée d'un ou de plusieurs écrans et d'une interface homme-machine 26.

L'interface homme-machine 26 comprend des moyens de pointage et de sélection d'éléments, par exemple un ensemble clavier-souris, un pavé tactile, une interface gestuelle 3D sans contact ou une combinaison de ces dispositifs.

Dans un mode de réalisation, l'interface homme-machine 26 est intégrée avec l'unité de visualisation 24 sous la forme d'un écran tactile.

Le dispositif programmable 22 est adapté à recevoir les images bidimensionnelles (fluoroscopiques et/ou angiographiques) acquises par le dispositif d'imagerie à rayons X et à les traiter selon un procédé d'aide au guidage d'un outil endovasculaire dans des structures vasculaires selon l'invention.

Dans un mode de réalisation, le dispositif programmable 22 est adapté à commander l'acquisition d'images bidimensionnelles (fluoroscopiques et/ou angiographiques).

Les images bidimensionnelles acquises pendant la phase d'intervention sont affichées sur l'unité de visualisation 24 pour aider à un guidage précis des outils endovasculaires à l'intérieur des structures vasculaires, en particulier des artères, du patient.

Les outils endovasculaires sont sélectionnés parmi un cathéter, un dispositif endovasculaire de type stent, un guide souple ou rigide, une endoprothèse ou un ballon.

La figure 2 illustre les principaux blocs d'un dispositif programmable 30 apte à mettre en œuvre le procédé d'aide au guidage d'un outil endovasculaire dans des structures vasculaires selon un mode de réalisation de l'invention.

Un dispositif programmable 30 apte à mettre en oeuvre l'invention, comprend un écran 32, analogue à l'unité de visualisation 24, une unité 34 de saisie des commandes d'un opérateur, par exemple un clavier, une souris, un pavé tactile ou une interface sans contact, analogue à l'unité 26, une unité centrale de traitement 36, ou CPU, apte à exécuter des instructions de programme informatique lorsque le dispositif 30 est mis sous tension. Le dispositif 30 comporte optionnellement un contrôleur 40, permettant d'envoyer des commandes et de sélectionner des éléments à distance.

Le dispositif 30 comporte également une unité de stockage d'informations 38, par exemple des registres, aptes à stocker des instructions de code exécutable permettant la mise en oeuvre de programmes comportant des instructions de code aptes à mettre en oeuvre le procédé selon l'invention. Les divers blocs fonctionnels du dispositif 30 décrits ci-dessus sont connectés via un bus de communication 42.

Le dispositif 30 est apte à recevoir des données d'image d'une source 44.

Dans un mode de réalisation, le dispositif 30 est adapté à coopérer avec un dispositif d'imagerie pour acquérir des images fluoroscopiques et/ou angiographiques. En particulier, le dispositif 30 est adapté à commander le dispositif d'imagerie pour acquérir une pluralité d'images angiographiques correspondant à la pluralité d'images fluoroscopiques.

Le procédé de l'invention est adapté à être mis en oeuvre par un dispositif programmable 30 tel un ordinateur intégré dans une salle d'intervention standard, ce qui permet de limiter les coûts d'équipement.

Dans un mode de réalisation, le procédé de l'invention est mis en oeuvre par des modules de code logiciel. Les instructions de code exécutable sont enregistrées sur un support lisible par ordinateur, par exemple un disque optique, un disque magnéto-optique, une mémoire ROM, une mémoire RAM, une mémoire non volatile (EPROM, EEPROM, FLASH, NVRAM), une carte magnétique ou optique. En variante les modules de code logiciel sont réalisés sous forme d'un composant logique programmable tel qu'un FPGA (pour *Field Programmable Gate Array),* ou encore sous forme de circuit intégré dédié tel qu'un ASIC (pour *Application Specific Integrated Circuit).*

Le dispositif programmable 30 est adapté à :
- obtenir une pluralité d'images fluoroscopiques partiellement superposées de la zone anatomique d'intérêt, et former une première image augmentée représentative d'un panorama osseux complet de ladite zone anatomique d'intérêt,
- obtenir une deuxième image augmentée comportant une représentation des structures vasculaires de ladite zone anatomique d'intérêt,
- obtenir une nouvelle image fluoroscopique, dite image fluoroscopique courante, d'une portion de la zone anatomique d'intérêt,
- recaler ladite image fluoroscopique courante par rapport à la première image augmentée, localiser et afficher sur l'unité de visualisation une zone d'image correspondant à l'image fluoroscopique courante dans la deuxième image augmentée.

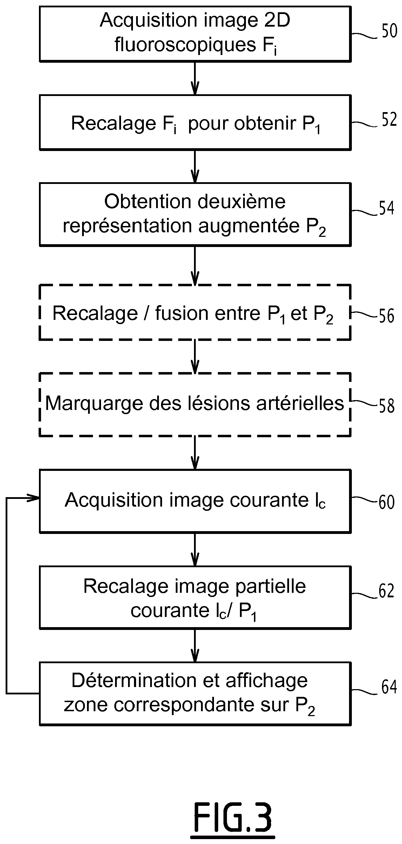

La figure 3 représente les principales étapes mises en œuvre dans un procédé d'aide au guidage d'un outil endovasculaire dans des structures vasculaires selon un mode de réalisation de l'invention, mises en oeuvre par un processeur 36.

Le procédé comporte, dans une première phase opératoire, une première étape 50 d'acquisition de N images fluoroscopiques bidimensionnelles d'un membre inférieur du patient, par exemple 3 ou 4 images successives acquises par translation du dispositif d'imagerie le long de l'axe fémoral.

Les images fluoroscopiques bidimensionnelles sont notées {F₁,..,Fᵢ,...F_{N}} et sont mémorisées sous forme d'images numériques. Deux images successives sont partiellement superposées, et présentent donc une zone de superposition rectangulaire de taille variable lorsque le dispositif est déplacé par l'opérateur, de préférence supérieure ou égale à une taille prédéterminée.

Par exemple, lorsque chaque image numérique est représentée sous forme d'une matrice bidimensionnelle de pixels, chaque pixel ayant une valeur numérique associée appelée intensité, la taille de l'image est définie par un nombre de lignes et un nombre de colonnes, une zone de superposition comporte un nombre de lignes et de colonnes formant une zone de surface donnée, de préférence supérieure ou égale à 20% de la surface totale de l'image.

L'étape 50 est suivie d'une étape 52 de recalage deux à deux des images fluoroscopiques bidimensionnelles pour former une première image augmentée, notée P₁, représentative d'un panorama osseux complet du membre inférieur représenté.

Le recalage d'images ou mise en correspondance spatiale consiste à placer en correspondance spatiale des structures anatomiques ou fonctionnelles présentes dans chacune des images. Par fusion successive des images recalées le long du membre inferieur, on obtient une image augmentée représentant la structure osseuse de la totalité du membre inférieur.

Dans le cas d'application présent, le recalage a pour objectif de réaliser une superposition précise des structures osseuses présentes dans la zone de superposition commune aux images successives.

Deux images successives peuvent présenter une légère rotation entre elles, en particulier lorsque l'opérateur déplace le dispositif d'imagerie. Le recalage 2D/2D consiste à optimiser deux translations et une rotation.

Les images fluoroscopiques bidimensionnelles acquises présentent un niveau de contraste relativement important grâce aux os. Il existe plusieurs méthodes de recalage rigide entre images bidimensionnelles (ou recalage 2D-2D) applicables pour des images comprenant des objets ayant un niveau de contraste relativement élevé.

Par exemple, un recalage automatique de type iconique peut être mis en oeuvre, en utilisant une mesure de similarité basée sur la différence de gradients entre les deux images, couplé à une stratégie d'optimisation de type descente de gradient ou optimiseur de Powell.

Suite à l'étape 52 de recalage 2D-2D est obtenue une première image augmentée P₁ représentant un panorama osseux du membre inférieur du patient. Comme expliqué ci-dessus l'image P₁ est obtenue à partir d'une pluralité d'images bidimensionnelles fluoroscopique acquises.

Lors d'une étape 54, au moins une deuxième image augmentée du membre inférieur du patient est obtenue. Cette deuxième image augmentée comporte une représentation des structures vasculaires du membre inférieur du patient.

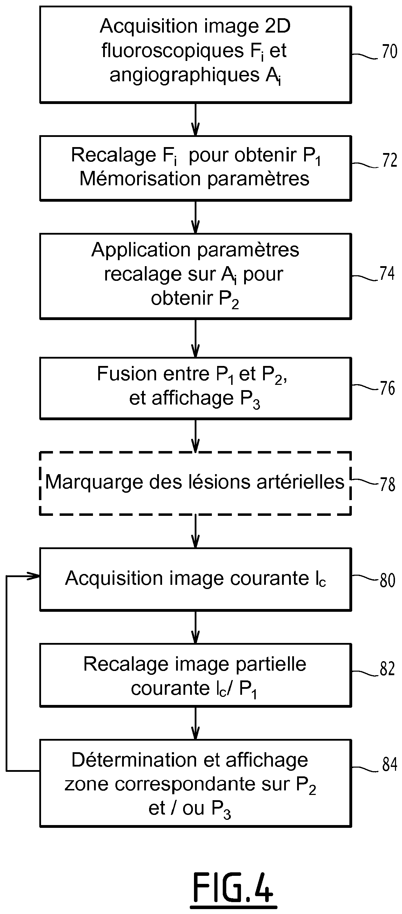

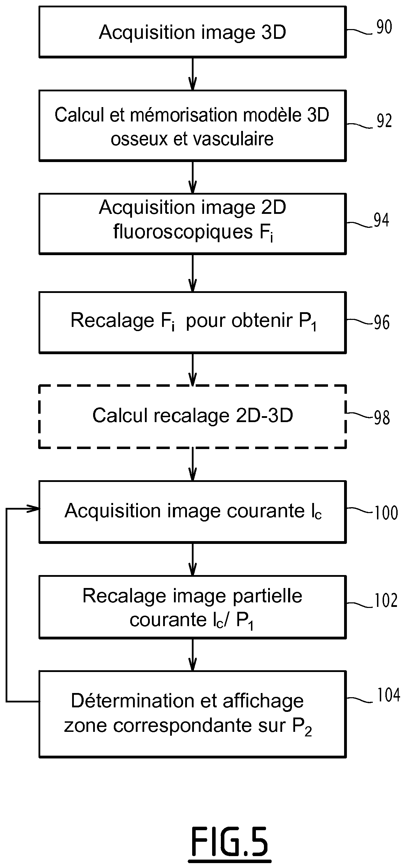

Deux modes de réalisation différents de l'étape 54 sont envisagés et seront décrits plus en détail ci-après en référence aux figures 4 et 5.

Dans un premier mode de réalisation, la deuxième image augmentée est une image bidimensionnelle représentative d'un panorama artériel du membre inférieur considéré, situé dans le même repère spatial que le panorama osseux P₁.

Le panorama artériel est obtenu à partir d'images angiographiques bidimensionnelles représentant les structures vasculaires du membre inférieur considéré. Les images angiographiques obtenues étant dans le dans le même repère spatial que les images fluoroscopiques, les paramètres de recalage obtenus à l'étape 50 sont directement appliqués aux images angiographiques pour obtenir le panorama artériel.

Dans un deuxième mode de réalisation, la deuxième image augmentée est une image tridimensionnelle des structures osseuse et vasculaire du membre inférieur considéré. Dans ce deuxième mode de réalisation, la deuxième image augmentée est obtenue dans une phase préalable, pré-opératoire, et mémorisée. Elle est par exemple calculée à partir d'images obtenues par des techniques d'acquisition comme par exemple la tomographie également appelée CT pour « computed tomography », l'imagerie par résonance magnétique (IRM), rehaussés par l'injection de produit de contraste pour mieux faire apparaitre les structures vasculaires.

Dans un troisième mode de réalisation, on utilise à la fois une deuxième image augmentée représentative d'un panorama artériel et une troisième image tridimensionnelle des structures osseuse et vasculaire du membre inférieur considéré.

Dans le cas d'obtention d'une image augmentée de type image tridimensionnelle pré-opératoire, l'étape 54 est suivie d'une étape optionnelle 56 de recalage entre les première et deuxième images augmentées.

Suite à l'étape 56, il est utile de représenter une image fusionnée P₃ du panorama osseux P₁ et de l'image augmentée P₂, qui servira pour la visualisation simultanée des structures osseuses et artérielles.

L'étape 56 ou l'étape 54 est suivie optionnellement d'une étape 58 de marquage, par l'opérateur du système, par exemple un médecin, des lésions artérielles observées sur la deuxième image augmentée P₂.

Dans une deuxième phase opératoire, une image fluoroscopique courante I_{c} est acquise à l'étape 60. La deuxième phase opératoire est une phase d'intervention effective, lors de laquelle un outil endovasculaire, sélectionné parmi un cathéter, un dispositif endovasculaire de type stent, un guide souple ou rigide, une endoprothèse ou un ballon, est introduit pour traiter le patient.

L'image fluoroscopique courante I_{c} est ensuite recalée par rapport à la première image augmentée P₁ représentative d'un panorama osseux du membre inférieur à l'étape 62.

Le recalage consiste ici à placer en correspondance spatiale des structures anatomiques osseuses présentes dans l'image fluoroscopique courante I_{c} avec des structures de la première image augmentée P₁.Le recalage utilisé ici est ainsi de type « template matching » du fait de l'appartenance de l'image courante I_{c} a une partie bien précise de la première image augmentée P₁.

De préférence, le recalage effectué est de type iconique, en utilisant une mesure de similarité basée sur la différence de gradients entre les deux images, une mesure de similarité basée sur l'information mutuelle, ou une combinaison des deux.

Enfin, à l'étape 64, une zone d'image correspondant à l'image fluoroscopique courante I_{c} est déterminée dans la deuxième image augmentée P₂ et/ou dans l'image fusionnée P₃, et affichée, grâce à des informations de recalage calculées et mémorisées entre la première image augmentée P₁ et la deuxième image augmentée P₂.

Avantageusement, la structure vasculaire locale est affichée en lien avec l'image fluoroscopique courante sans nécessiter une nouvelle acquisition d'image angiographique, et par conséquent sans nécessité d'injecter un supplément de produit de contraste.

Avantageusement, dans le cas où le marquage des lésions a été effectué lors de l'étape 58, les lésions sont localisées précisément par rapport à l'image fluoroscopique courante grâce aux recalages effectués entre l'image fluoroscopique courante et la première image augmentée P₁ et grâce au recalage entre la première image augmentée P₁ et la deuxième image augmentée P₂.

Les étapes 60 à 64 sont itérées sensiblement en temps réel autant que nécessaire durant l'intervention, notamment pour chaque lésion à traiter sur le membre inférieur.

La figure 4 illustre plus en détail un premier mode de réalisation de l'invention, dans lequel la deuxième image augmentée P₂ est un panorama artériel du membre inférieur du patient.

Dans ce premier mode de réalisation, à l'étape d'acquisition d'images 70, à chaque position d'acquisition d'images du dispositif d'imagerie, on acquiert une image fluoroscopique Fᵢ et une image angiographique Aᵢ. Ainsi, il y a une correspondance spatiale initiale entre ces images. En d'autres termes, les images sont acquises dans le même repère spatial.

L'étape 72 de recalage est analogue à l'étape 52 précédemment décrite. Les paramètres de recalage rigide 2D-2D entre images fluoroscopiques successives sont mémorisés, et appliqués ensuite à l'étape 74 aux images angiographiques Aᵢ pour obtenir la deuxième image augmentée P₂.

Ainsi, avantageusement, les paramètres de recalage sont calculés sur les images fluoroscopiques qui présentent un bon contraste grâce à la présence des structures osseuses. Grâce à l'acquisition des images fluoroscopiques et angiographiques en correspondance spatiale, un panorama des structures vasculaires correspondant au panorama osseux est obtenu aisément.

Les première et deuxième images augmentées sont fusionnées à l'étape de fusion 76 dans une image fusionnée P₃ faisant apparaître à la fois les structures osseuses et vasculaires de la totalité du membre inférieur étudié. La fusion comprend par exemple une somme pondérée par des coefficients de pondération, pixel par pixel, des images augmentées P₁ et P₂, les coefficients de pondération associés aux pixels respectifs étant choisis en fonction de l'intensité initiale des images.

L'image P₃ est affichée, par exemple à côté de la deuxième image augmentée P₂. Bien évidemment, des variantes d'affichage sont envisageables.

A l'étape optionnelle de marquage 78, l'opérateur ajoute des marqueurs, dont la position est enregistrée, pour marquer les lésions artérielles à traiter.

Les marqueurs sont de préférence ajoutés sur la deuxième image augmentée P₂ représentative du panorama des structures vasculaires, et reportés précisément sur l'image fusionnée P₃. Les marqueurs sont par exemple représentés par des traits de couleur superposés sur la ou les images affichées.

En variante, les marqueurs sont ajoutés sur la deuxième image augmentée P₂ avant l'étape 76 de calcul de l'image fusionnée P₃, et sont reportés sur l'image fusionnée affichée.

Selon une autre variante, les marqueurs sont ajoutés directement sur l'image fusionnée P₃.

L'étape 80 d'acquisition d'image fluoroscopique courante I_{c} est analogue à l'étape 60 décrite précédemment.

L'image fluoroscopique courante est ensuite recalée par rapport à la première image augmentée P₁ à l'étape 82, analogue à l'étape 62 précédemment décrite.

Ensuite, à l'étape 84, les structures vasculaires correspondant à l'image fluoroscopique courante sont relocalisées dans la deuxième image augmentée P₂ et/ou dans l'image fusionnée P₃, et sont affichées.

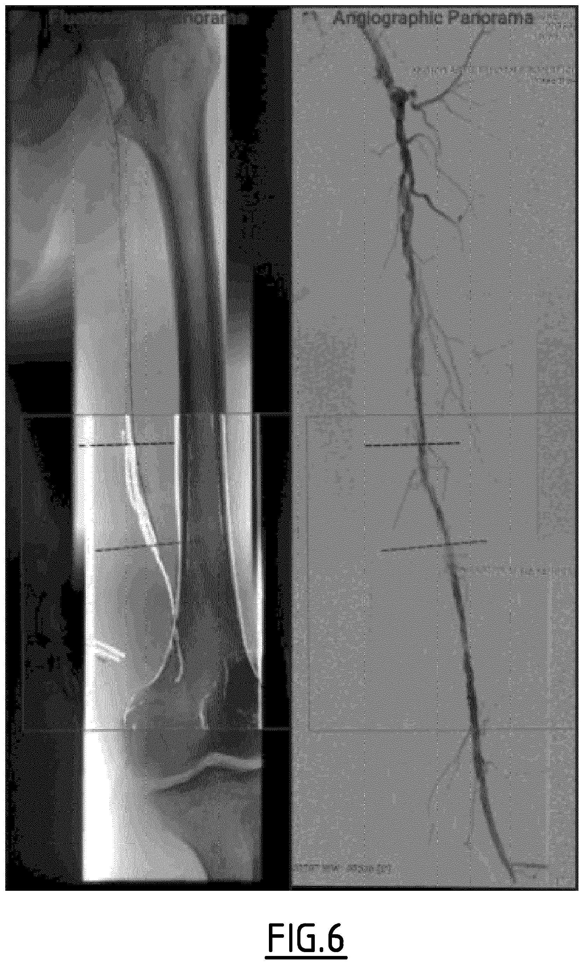

Par exemple, dans un mode de réalisation, un cadre indiquant la zone correspondant à l'image fluoroscopique courante est affiché, à la fois sur l'image augmentée P₂ et l'image fusionnée P₃, qui sont affichées en parallèle, ainsi que les marqueurs précédemment positionnés, comme illustré dans l'exemple de la figure 6.

Dans la figure 6, la partie de gauche représente une image fusionnée, comportant le panorama osseux de la première image augmentée fusionné avec l'image fluoroscopique courante, ainsi que des marqueurs de délimitation d'une lésion artérielle. En parallèle, sur la partie droite de la figure, la deuxième image augmentée représentative du panorama artériel est représentée, avec également des marqueurs de délimitation de la même lésion artérielle.

De plus, selon une variante, une image représentative de l'image fluoroscopique courante avec en superposition la structure angiographique correspondante, et, le cas échéant, avec les marqueurs préalablement enregistrés, est affichée, avec une meilleure résolution d'affichage que la résolution de la zone affichée sur l'image augmentée P₂ ou sur l'image P₃.

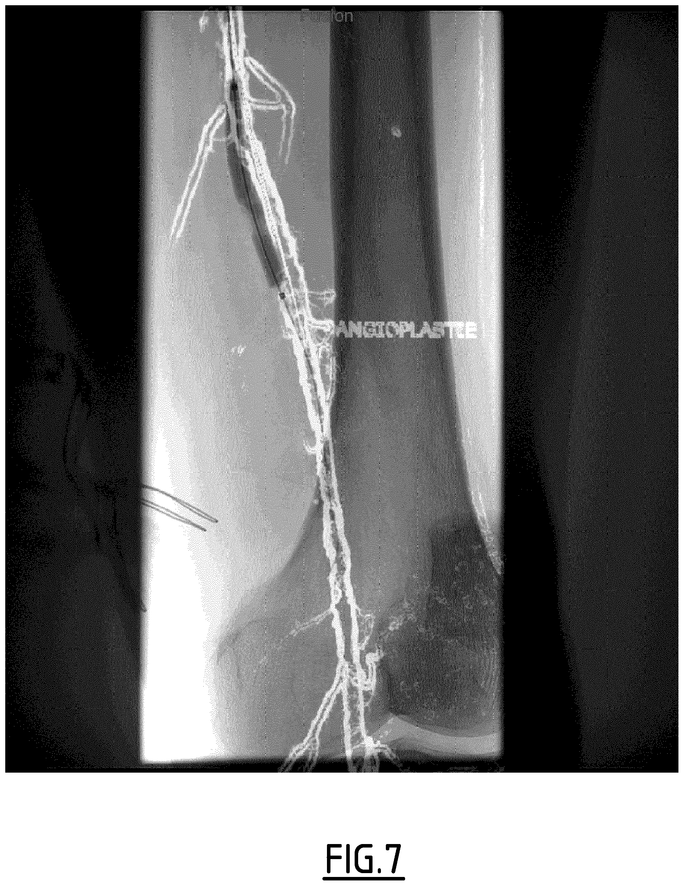

La figure 7 illustre un exemple d'affichage d'une image fluoroscopique courante, avec en superposition la structure angiographique correspondante issue de la deuxième image augmentée.

Ainsi, avantageusement, les lésions artérielles à traiter sont relocalisées, en lien avec l'image fluoroscopique courante et avec les images augmentée P₂ et fusionnée P₃ du membre inférieur, ce qui permet d'aider au guidage de l'outil endovasculaire introduit en cours d'intervention sans nouvelle acquisition d'image angiographique.

La figure 5 illustre plus en détail un deuxième mode de réalisation de l'invention, dans lequel la deuxième image augmentée P₂ est une image 3D représentative des structures osseuses et vasculaires du membre inférieur du patient.

Dans ce mode de réalisation, la deuxième image augmentée P₂ est obtenue et mémorisée dans une phase préopératoire. Cette image tridimensionnelle est par exemple calculée à partir d'images obtenues à l'étape pré-opératoire 90 par des techniques d'acquisition comme par exemple la tomographie également appelée CT pour « computed tomography », ou l'imagerie par résonance magnétique (IRM), rehaussés par l'injection de produit de contraste pour mieux faire ressortir les structures vasculaires.

A partir des images obtenues une image 3D est calculée et mémorisée à l'étape pré-opératoire 92. Il existe diverses méthodes connues de calcul d'une telle image. Par exemple, en travaillant sur l'image CT obtenue avec injection de produit de contraste, une segmentation automatique des structures artérielles peut être utilisée pour créer un modèle anatomique tridimensionnel du patient. Un algorithme de segmentation semi-automatique de type coupe de graphe peut être utilisé.

Par exemple, un tel modèle anatomique tridimensionnel virtuel peut comprendre les volumes segmentés de l'artère fémorale, des artères iliaques internes, des hanches et du fémur.

En phase opératoire, le procédé comporte une étape 94 d'acquisition d'images bidimensionnelles fluoroscopiques, analogue à l'étape 50 précédemment décrite, suivie d'une étape de recalage 96 pour former la première image augmentée P₁, analogue à l'étape 52 précédemment décrite.

Un recalage 2D-3D rigide est ensuite appliqué à l'étape 98, et des paramètres permettant la mise en correspondance de la première image augmentée P₁ et la deuxième image augmentée tridimensionnelle sont mémorisés.

Toute méthode de recalage 2D-3D rigide connue est applicable. Par exemple, un recalage semi-automatique de type iconique est mis en oeuvre, dans lequel l'initialisation d'une partie des points utilisés pour le recalage est effectuée manuellement, ce qui permet de faire correspondre grossièrement les deux images à recaler, et le recalage automatique est ensuite lancé par la suite pour affiner le résultat.

L'étape 98 est suivie des étapes d'acquisition 100 d'une image bidimensionnelle fluoroscopique courante, représentative d'une portion du membre inférieur considéré, analogue à l'étape 60 précédemment décrite, puis d'une étape 102 de recalage par rapport à la première image augmentée P₁, analogue à l'étape 62 déjà décrite.

Enfin, lors de l'étape 104, une zone correspondant à l'image courante est déterminée dans l'image tridimensionnelle P₂, en appliquant les paramètres de mise en correspondance 2D-3D entre la première image augmentée P₁ et l'image tridimensionnelle P₂ calculés et mémorisés à l'étape 98.

En variante, un recalage 2D-3D est effectué à l'étape 104.

Enfin, une zone correspondant à l'image fluoroscopique courante est affichée sur l'image tridimensionnelle P₂.

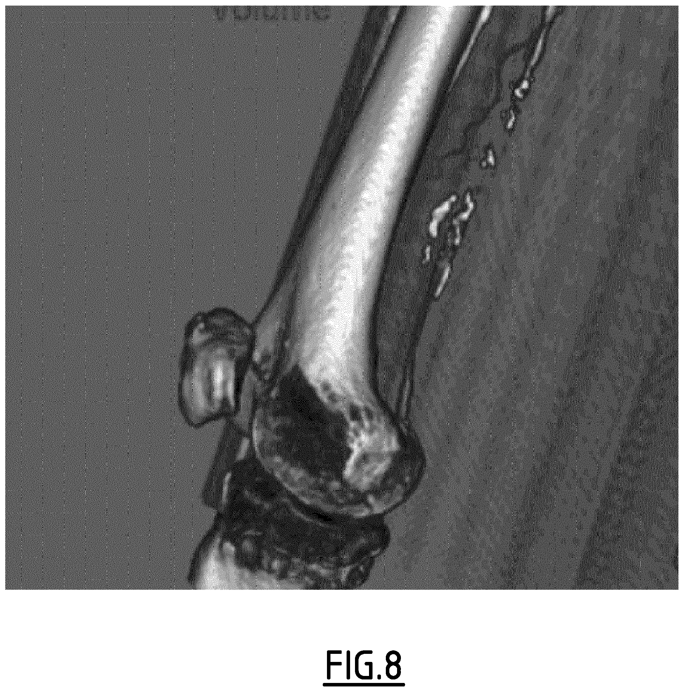

La figure 8 illustre un exemple d'image 3D représentative des structures osseuses et vasculaires d'une portion de membre inférieur, superposée avec une image fluoroscopique courante.

Bien évidemment, des variantes de détail de la mise en oeuvre des étapes ci-dessus, à la portée de l'homme du métier, sont tout à fait envisageables.

Dans un troisième mode de réalisation, les deux premiers modes de réalisation sont combinés. La deuxième image augmentée P₂ représentative d'un panorama artériel est calculée et affichée, comme dans le premier mode de réalisation. De plus, une autre image augmentée P'₂, qui est l'image tridimensionnelle représentative des structures osseuses et vasculaires du membre inférieur, est également calculée et affichée comme dans le deuxième mode de réalisation.

La zone correspondant à l'image fluoroscopique courante est déterminée et affichée sur chacune des images augmentées.

Ainsi, le procédé permet l'affichage, dans la deuxième phase opératoire, d'une zone correspondant à l'image fluoroscopique courante à la fois dans l'image augmentée correspondant au panorama artériel, et dans l'image augmentée tridimensionnelle.

Avantageusement, l'opérateur dispose d'informations sur les lésions artérielles, ainsi que d'informations complémentaires sur le patient, par exemple la localisation des calcifications visible uniquement dans l'image 3D.

Dans tous les modes de réalisation, il n'est pas nécessaire d'injecter du produit de contraste et d'acquérir de nouvelles images angiographiques correspondant aux images fluoroscopiques courantes, donc la quantité de produit de contraste à injecter au patient est diminuée par rapport aux interventions conventionnelles.

De plus, avantageusement, plus d'informations sont affichées, ce qui permet d'améliorer l'aide apportée dans le cadre d'interventions endovasculaires.

## Revendications

1. Système d'aide au guidage d'un outil endovasculaire dans des structures vasculaires d'une zone anatomique d'intérêt d'un patient, comprenant un dispositif d'imagerie apte à acquérir des images bi-dimensionnelle de portions du corps d'un patient, un dispositif programmable et une unité de visualisation :
- le dispositif d'imagerie étant adapté à acquérir une pluralité d'images fluoroscopiques (Fᵢ) partiellement superposées de la zone anatomique d'intérêt, et le dispositif programmable étant configuré pour former une première image augmentée (P₁) représentative d'un panorama osseux complet de ladite zone anatomique d'intérêt,
- le dispositif programmable étant configuré pour commander le dispositif d'imagerie pour acquérir une pluralité d'images angiographiques correspondant à ladite pluralité d'images fluoroscopiques et pour former une deuxième image augmentée (P₂),
- le dispositif d'imagerie étant configuré pour acquérir une nouvelle image fluoroscopique, dite image fluoroscopique courante (l_{c}), d'une portion de la zone anatomique d'intérêt,
- le dispositif programmable étant configuré pour recaler ladite image fluoroscopique courante (l_{c}) par rapport à la première image augmentée (P₁), localiser et afficher sur l'unité de visualisation une zone d'image correspondant à l'image fluoroscopique courante dans la deuxième image augmentée (P₂),
ledit système étant **caractérisé en ce que** ladite deuxième image augmentée (P₂) est une image bidimensionnelle représentative d'un panorama artériel de la zone anatomique d'intérêt.

2. Système selon la revendication 1, dans lequel le dispositif programmable est configuré pour afficher des marqueurs délimitant des lésions artérielles observées sur la deuxième image augmentée (P₂).

3. Système selon la revendication 2, dans lequel le dispositif programmable est configuré pour commander le dispositif d'imagerie pour effectuer l'acquisition d'une pluralité d'images angiographiques sensiblement en parallèle de l'acquisition d'une pluralité d'images fluoroscopiques partiellement superposées, le dispositif d'imagerie étant à une même position spatiale pour l'acquisition d'une image fluoroscopique et d'une image angiographique correspondante.

4. Système selon l'une des revendications 2 ou 3, dans lequel le dispositif programmable est configuré pour effectuer une fusion de la première image augmentée (P₁) et de la deuxième image augmentée (P₂) et un affichage sur l'unité de visualisation de l'image fusionnée.

5. Système selon la revendication 4, dans lequel le dispositif programmable est configuré pour en outre afficher sur l'unité de visualisation une zone d'image correspondant à l'image fluoroscopique courante dans l'image fusionnée.

6. Système selon la revendication 5 dans lequel le dispositif programmable est configuré pour, dans une phase préopératoire, coopérer avec le dispositif d'imagerie pour obtenir une troisième image augmentée représentative des structures osseuses et vasculaires de la zone anatomique d'intérêt, et, après localisation d'une zone d'image correspondant à l'image fluoroscopique courante dans la deuxième image augmentée, localiser et afficher une zone d'image correspondant à l'image fluoroscopique courante dans la troisième image augmentée.

7. Programme d'ordinateur comportant des instructions pour mettre en oeuvre les étapes d'un procédé d'aide au guidage d'un outil endovasculaire dans des structures vasculaires lors de l'exécution du programme par un processeur d'un dispositif programmable, comportant des étapes de :
- acquisition (50) d'une pluralité d'images fluoroscopiques bidimensionnelles (Fᵢ) partiellement superposées de la zone anatomique d'intérêt du patient, et formation (52) d'une première image augmentée (P₁) représentative d'un panorama osseux complet de ladite zone anatomique d'intérêt,
- acquisition d'une pluralité d'images angiographiques correspondant à ladite pluralité d'images fluoroscopiques, et formation (54) d'une deuxième image augmentée (P₂), ladite deuxième image augmentée (P₂) étant une image bidimensionnelle représentative d'un panorama artériel de la zone anatomique d'intérêt,
- acquisition (60) d'une nouvelle image fluoroscopique bidimensionnelle, dite image fluoroscopique courante (l_{c}), d'une portion de ladite zone anatomique d'intérêt,
- recalage (62) de l'image fluoroscopique courante (l_{c}) par rapport à la première image augmentée (P₁),
- localisation et affichage (64) d'une zone d'image correspondant à l'image fluoroscopique courante dans la deuxième image augmentée (P₂).

8. Programme d'ordinateur selon la revendication 7, comportant la mise en oeuvre d'une étape d'affichage de marqueurs délimitant des lésions artérielles observées sur la deuxième image augmentée (P₂).

## Patentansprüche

1. System zur Unterstützung der Führung eines endovaskulären Instruments in vaskulären Strukturen einer anatomischen Region von Interesse eines Patienten, umfassend eine Bildgebungsvorrichtung, die dazu geeignet ist, zweidimensionale Bilder von Abschnitten des Körpers eines Patienten zu erfassen, eine programmierbare Vorrichtung und eine Visualisierungseinheit:
- wobei die Bildgebungsvorrichtung dazu geeignet ist, eine Vielzahl von teilweise überlagerten fluoroskopischen Bildern (Fᵢ) der anatomischen Region von Interesse zu erfassen, und die programmierbare Vorrichtung dazu eingerichtet ist, ein erstes vergrößertes Bild (P₁) zu bilden, das für ein komplettes Knochenpanorama der genannten anatomischen Region von Interesse repräsentativ ist,
- wobei die programmierbare Vorrichtung dazu eingerichtet ist, die Bildgebungsvorrichtung dazu zu steuern, eine Vielzahl von angiografischen Bildern zu erfassen, die der genannten Vielzahl von fluoroskopischen Bildern entsprechen, und um ein zweites vergrößertes Bild (P₂) zu bilden,
- wobei die Bildgebungsvorrichtung dazu eingerichtet ist, ein neues fluoroskopisches Bild, das als aktuelles fluoroskopisches Bild (I_{c}) bezeichnet wird, eines Abschnitts der anatomischen Region von Interesse zu erfassen,
- wobei die programmierbare Vorrichtung dazu eingerichtet ist, das genannte aktuelle fluoroskopische Bild (I_{c}) in Bezug auf das erste vergrößerte Bild (P₁) zu registrieren, und eine Bildregion, die dem aktuellen fluoroskopischen Bild entspricht, zu lokalisieren und auf der Visualisierungseinheit in dem zweiten vergrößerten Bild (P₂) anzuzeigen,
wobei das genannte System **dadurch gekennzeichnet ist, dass** das genannte zweite vergrößerte Bild (P₂) ein zweidimensionales Bild ist, das für ein Arterienpanorama der anatomischen Region von Interesse repräsentativ ist.

2. System nach Anspruch 1, wobei die programmierbare Vorrichtung dazu eingerichtet ist, Marker anzuzeigen, welche auf dem zweiten vergrößerten Bild (P₂) beobachtete Arterienläsionen umgrenzen.

3. System nach Anspruch 2, wobei die programmierbare Vorrichtung dazu eingerichtet ist, die Bildgebungsvorrichtung dazu zu steuern, die Erfassung einer Vielzahl von angiografischen Bildern im Wesentlichen parallel zur Erfassung einer Vielzahl von teilweise überlagerten fluoroskopischen Bildern zu bewirken, wobei sich die Bildgebungsvorrichtung zur Erfassung eines fluoroskopischen Bilds und eines entsprechenden angiografischen Bilds in derselben Raumposition befindet.

4. System nach einem der Ansprüche 2 oder 3, wobei die programmierbare Vorrichtung dazu eingerichtet ist, eine Fusion des ersten vergrößerten Bilds (P₁) und des zweiten vergrößerten Bilds (P₂) und eine Anzeige des fusionierten Bilds auf der Visualisierungseinheit zu bewirken.

5. System nach Anspruch 4, wobei die programmierbare Vorrichtung dazu eingerichtet ist, ferner auf der Visualisierungseinheit eine Bildregion, die dem aktuellen fluoroskopischen Bild entspricht, in dem fusionierten Bild anzuzeigen.

6. System nach Anspruch 5, wobei die programmierbare Vorrichtung dazu eingerichtet ist, in einer präoperativen Phase mit der Bildgebungsvorrichtung zusammenzuwirken, um ein drittes vergrößertes Bild zu erhalten, das für Knochen- und vaskuläre Strukturen der anatomischen Region von Interesse repräsentativ ist, und, nach der Lokalisierung einer Bildregion, die dem aktuellen flouroskopischen Bild entspricht, in dem zweiten vergrößerten Bild, eine Bildregion in dem dritten vergrößerten Bild zu lokalisieren und anzuzeigen, die dem aktuellen flouroskopischen Bild entspricht.

7. Computerprogramm, umfassend Instruktionen zur Umsetzung der Schritte eines Verfahrens zur Unterstützung der Führung eines endovaskulären Instruments in vaskulären Strukturen während der Ausführung des Programms durch einen Prozessor einer programmierbaren Vorrichtung, umfassend die Schritte:
- Erfassen (50) einer Vielzahl von teilweise überlagerten zweidimensionalen fluoroskopischen Bildern (Fᵢ) der anatomischen Region von Interesse des Patienten, und Bilden (52) eines ersten vergrößerten Bilds (P₁), das für ein komplettes Knochenpanorama der genannten anatomischen Region von Interesse repräsentativ ist,
- Erfassen einer Vielzahl von angiografischen Bildern, die der genannten Vielzahl von fluoroskopischen Bildern entsprechen, und Bilden (54) eines zweiten vergrößerten Bilds (P₂), wobei das genannte zweite vergrößerte Bild (P₂) ein zweidimensionales Bild ist, das für ein Arterienpanorama der genannten anatomischen Region von Interesse repräsentativ ist,
- Erfassen (60) eines neuen zweidimensionalen fluoroskopischen Bilds, das als aktuelles fluoroskopisches Bild (I_{c}) bezeichnet wird, eines Abschnitts der genannten anatomischen Region von Interesse,
- Registrieren (62) des aktuellen fluoroskopischen Bilds (I_{c}) in Bezug auf das erste vergrößerte Bild (P₁),
- Lokalisieren und Anzeigen (64) einer Bildregion, die dem aktuellen fluoroskopischen Bild entspricht, in dem zweiten vergrößerten Bild (P₂).

8. Computerprogramm nach Anspruch 7, umfassend die Durchführung eines Schritts der Anzeige von Markern, welche auf dem zweiten vergrößerten Bild (P₂) beobachtete Arterienläsionen umgrenzen.

## Claims

1. System for assistance in guiding an endovascular instrument in vascular structures of an anatomical region of interest of a patient, comprising an imaging device suitable for capturing two-dimensional images of parts of the body of a patient, a programmable device and a viewing unit:
- the imaging device being suitable for capturing a plurality of partially superposed fluoroscopic images (Fᵢ) of the anatomical region of interest, and the programmable device being configured to form a first augmented image (P₁) representative of a complete panorama of the bones of said anatomical region of interest,
- the programmable device being configured to control the imaging device in order to capture a plurality of angiographic images corresponding to said plurality of fluoroscopic images and to form a second augmented image (P₂),
- the imaging device being configured to capture a new fluoroscopic image, called current fluoroscopic image (I_{c}), of a part of the anatomical region of interest,
- the programmable device being configured to register said current fluoroscopic image (I_{c}) with respect to the first augmented image (P₁), to locate and display, on the viewing unit an image region corresponding to the current fluoroscopic image in the second augmented image (P₂),
said system being **characterised in that** said second augmented image (P₂) is a two-dimensional image representative of an arterial panorama of the anatomical region of interest.

2. System according to claim 1, wherein the programmable device is configured to display markers that delimit arterial lesions observed on the second augmented image (P₂) .

3. System according to claim 2, wherein the programmable device is configured to control the imaging device in order to capture a plurality of angiographic images substantially in parallel with the capturing of a plurality of partially superposed fluoroscopic images, the imaging device being at the same spatial position for the capturing of a fluoroscopic image and of a corresponding angiographic image.

4. System according to one of claims 2 or 3, wherein the programmable device is configured to carry out a merger of the first augmented image (P₁) and of the second augmented image (P₂) and a displaying on the viewing unit of the merged image.

5. System according to claim 4, wherein the programmable device is further configured to display on the viewing unit an image region corresponding to the current fluoroscopic image in the merged image.

6. System according to claim 5 wherein the programmable device is configured to, in a pre-operative phase, cooperate with the imaging device in order to obtain a third augmented image representative of the bone and vascular structures of the anatomical region of interest, and, after locating an image region corresponding to the current fluoroscopic image in the second augmented image, locating and displaying an image region corresponding to the current fluoroscopic image in the third augmented image.

7. Computer program comprising instructions for implementing the steps of a method for assistance in guiding an endovascular instrument in vascular structures during the execution of the program by a processor of a programmable device, comprising steps of:
- capturing (50) a plurality of partially superposed two-dimensional fluoroscopic images (Fᵢ) of the anatomical region of interest of the patient, and forming (52) a first augmented image (P₁) representative of a complete panorama of the bones of said anatomical region of interest,
- capturing a plurality of angiographic images corresponding to said plurality of fluoroscopic images, and forming (54) a second augmented image (P₂), said second augmented image (P₂) being a two-dimensional image representative of an arterial panorama of the anatomical region of interest,
- capturing (60) a new two-dimensional fluoroscopic image, called the current fluoroscopic image (I_{c}), of a portion of said anatomical region of interest,
- registering (62) the current fluoroscopic image (I_{c}) with respect to the first augmented image (P₁),
- locating and displaying (64) an image region corresponding to the current fluoroscopic image in the second augmented image (P₂).

8. Computer program according to claim 7, comprising the implementing of a step of displaying markers that delimit arterial lesions observed on the second augmented image (P₂).
